# EUROPEAN PATENT APPLICATION

(11) **EP 4 454 614 A2**
(43) Date of publication of application: **30.10.2024**
(21) Application number: 24193714.3
(22) Date of filing: 21.11.2019
(51) Int. Cl.: A61F 2/28

(54) **CRANIAL-MAXILLOFACIAL IMPLANT**

(30) Priority: 22.11.2018 BE 201805820
(62) Divisional of application: 19804747.4
(71) Applicant: Cerhum SA, 4000 Liège (BE)
(72) Inventor: NOLENS, Grégory, 4680 Oupeye (BE); BREULS DE TIECKEN, Thibaut, 6990 Hotton (Fronville) (BE)
(74) Representative: Gevers Patents

(57) **Abstract**

Implant made of a biocompatible ceramic of synthetic origin obtained by additive manufacturing, wherein said implant has a material density by volume ranging from 20% to 100%; wherein said implant comprises cavities delimited at least partially by side walls defining cavity sections, said cavity sections each having an extension such that it is possible to fit each of said cavity sections in a circle having a diameter ranging from 0.3 mm to 1.2 mm; and wherein said side walls delimiting at least partially the cavities are defined by a triply periodic minimal surface.

## Description

### Technical field

According to a first aspect, the invention relates to a cranial-maxillofacial implant. According to a second aspect, the invention relates to a method for manufacturing a cranial-maxillofacial implant.

### State of the art

Implants and methods exist to repair gaps, malformations or bone defects of the cranium. A bone hollowness of the cranium can be remedied manually, by using cement that is applied directly on the hollowness to be treated, or by using a custom-built implant. Malformations or defects of the face of an individual, which are often the cause of functional disorders, can be treated by a graft or by using a synthetic implant.

The implants used to fill and repair bones of the cranium or of the face are implanted under the skin, and require the use of biocompatible materials to avoid rejection reactions. Biocompatible materials used to perform facial implants can come from the individual in whom the implant is intended to be transplanted, i.e. after collecting and preparing bone or cartilage fragments for example. Materials of animal origin can also be used; these must be prepared by mechanical and chemical processes. Metallic, plastic or ceramic biocompatible materials can also be used.

A biocompatible implant provides good compatibility with the organism in which it is implanted, thereby ensuring that the organism does not reject it. However, biocompatible materials have disadvantages in the longer term, owing to the development of scar tissue and/or an inflammatory response to the foreign body in the area surrounding these implants, which then require additional surgical procedures. To remedy this disadvantage, biocompatible materials can also be bioresorbable, i.e. they can be assimilated by the organism, so as to be colonised and cause the regeneration of the bone with which the implant is positioned to come into contact.

Document EP 2 014 256 A1 describes a porous material manufactured with biocompatible ceramic for the repair of bone defects. The material described in the document provides a biocompatible material for the regeneration of the injured bone.

One of the problems of biocompatible materials suitable for the regeneration of injured bone is that they are relatively fragile, in particular porous implants which are brittle, making them vulnerable to possible shocks. Furthermore, osseointegration cannot be guaranteed for the entire implant, rendering portions that have not undergone osseointegration vulnerable to possible shocks.

### Summary of the invention

According to a first aspect, one of the purposes of the invention is to provide an implant that guarantees the best mechanical properties from the moment of its implantation and the best integration of the osseointegrable parts of the implant. Another purpose of the invention is to provide an implant that features good integration properties, in order to reduce the risk of infection associated with porous parts of the implant. For this purpose, the inventors propose an implant made of a biocompatible ceramic of synthetic origin obtained by additive manufacturing and comprising:
- a dense portion, with a material density by volume greater than 70%, preferably greater than 80%, and even more preferably between 95% and 99%, and for example equal to 99%, and
- a porous portion connected to said dense portion by a connection zone, said porous portion comprising an average macroporosity characterised by:
   ∘ an average material density determined for the entire volume of said porous portion of between 30% and 70% by volume;
   ∘ cavities delimited at least partially by side walls defining cavity sections, said cavity sections each having an extension such that it is possible to fit each of said cavity sections in a circle having a diameter ranging from 0.3 mm to 1.2 mm;
   said dense portion and said porous portion define at least partially an external surface of the implant;
   said cavities open onto said external surface.

The invention provides a biocompatible, biointegrable and potentially resorbable, and even bioresorbable implant. The invention provides a rigid implant thanks to the biocompatible ceramic of synthetic origin, and in particular thanks to the presence of the dense portion. The implant enables osseointegration with the bone next to which it is implanted thanks to the macroporosity of the porous portion. The macroporosity of the porous portion enables a faster osseointegration that features better mechanical properties than the osseointegration observed with implants according to the state of the art. The invention provides an implant with the advantages of a ceramic implant and with the advantages of a biointegrable and potentially bioresorbable implant. The invention provides a synthetic implant that has better osseointegration properties than that observed with the implants according to the state of the art. The invention provides an implant with a macroporosity enabling an osteoconduction of the bone with which it is contact when it is implanted in the macroporosities, ensuring quick anchoring of the implant in the bone. Furthermore, the implant enables the quick regrowth of the tissues with which it is in contact, in order to also provide a good support of the implant in the tissue in which it is implanted.

Osseointegration is a stable anchoring of the implant by direct contact of the bone and the implant. In the field of implants relating to the bones of the head, in particular of the cranium, the face and/or the jawbones, osseointegration is the method by which the highest successful implant rate is achieved. The implant according to the invention is therefore highly suitable for cranial implantation, thanks to its porous structure providing an excellent anchoring by osseointegration, and thanks to its dense portion, that provides the implant with its good mechanical properties. According to the state of the art, good anchoring by osseointegration, and good mechanical properties, are known to be antagonistic properties.

The advantage of an implant with a macroporosity in which the diameter of the pores and the distribution of the pores are well controlled is that it enables an osseointegration and possibly a bioresorbtion of the implant that are faster and more homogeneous. Furthermore, this allows the proper integration of the implant by the bone with which the implant is positioned to be in contact. It also provides results in terms of the mechanical properties, and in terms of reduced implant rejection risks that are more satisfactory when using an implant according to the invention. For example, the invention provides better results than implants with a metal base, a polymer base, or with a base made of composite materials of the ceramic/polymer type. The implant according to the invention yields results that are very similar to, and even better than the results achieved with autotransplantation.

Another advantage of the invention with respect to the state of the art is that it provides a distribution of the porous portion and of the dense portion such that the porous portion enables quick and high-quality osseointegration of the implant by the bone, without the zones of the porous portion being colonised, or without these zones of the porous portion being colonised over a relatively long period. This is made possible by a positioning of the porosity that is sufficiently deep to ensure the proper osseointegration or incorporation of the implant by the bone, but without the cavities quickly undergoing osseointegration, in which case such cavities could constitute a source of infection. Indeed, the osseointegration being in large part ensured by osteoinduction, and possibly by osteoconduction from the bone with which the implant is in contact, a relatively shallow macroporosity is necessary to ensure the proper osseointegration of the implant. A suitable porosity depth to achieve satisfactory osseointegration is of several tens of micrometres to several millimetres, for example 10 µm, 20 µm, 50 µm, 100 µm, 500 µm, 1 mm to 2 mm, 3 mm, 5 mm, 8 mm, 10 mm, 15 mm. A porosity depth can be measured by introducing, for example, a rigid or flexible measuring gauge, to reach the dense portion from the external surface of the porous portion. The dense portion is designed so as to provide the mechanical properties of the implant. Thus, the distribution of the dense portions and the porous portions is what enables the invention to provide an implant having good mechanical properties and osseointegration properties that allow a good integration of the implant, such as an original bone, on its implantation site.

The implant according to the invention is manufactured by additive manufacturing and preferably by 3D printing. This manufacturing method provides a controlled and interconnected macroporosity, i.e. properly distributed throughout the implant, with cavities featuring regular and uniform sections that are connected to one another. Furthermore, this manufacturing method controls the macroporosity of the implant, and the roughness of the outer walls or of the walls delimiting the cavities. Additive manufacturing enables excellent control of macroporosity and of the overall shape of the implant, without having to resort to machining operations on the implant following its manufacturing. Typically, the control of macroporosity and of the interconnections that is made possible by additive manufacturing cannot be achieved with an injection method or a traditional machining process. An injection process is, for example, a process whereby the macroporosity is formed with a sacrificial material that is removed after the hardening of the matrix of the implant around the sacrificial material, revealing a porous material. The advantage of having an implant with dimensions and properties of the porous portion adapted to the properties of the bone with which it is positioned to be in contact enables good contact of the bone with the porous portion or portions of the implant. This is very important to ensure quick osseointegration, thereby enabling a proper anchoring of the implant on the bone. This is partly made possible thanks to good imagery of the bone requiring the implant, i.e. a good delimitation of its contours in a three-dimensional space. Furthermore, this requires a manufacturing technique achieving an optimal distribution of the dense and porous portions, and dense portions having a cavity depth enabling optimal osseointegration, while excluding a maximum of infection risks due to poorly controlled macroporosity.

Preferably, the porosity of the implant is uniform, i.e. it is characterised by a single shape. For example, the term "uniform" means that the cavities that have a given cavity section is distributed regularly throughout the implant and that said cavity sections are controlled in terms of their size. The controlled cavity sections have predefined and preferably equal cavity sections. Preferably, the porosity is homogeneous, i.e. for a given volume and zone, or for several given volumes and zones, the porosity is evenly distributed throughout the volume. Preferably, the porosity is defined by a regular distribution of the pores throughout a given zone or volume. Preferably, the porosity of the implant is connected, i.e. it is possible to follow a route connecting the porosities without having to exit the implant. For example, a cross-section along a plane of a porous portion with a uniform porosity features a pattern, the pattern being associated with a cavity or with a cavity featuring an interconnection, this being true when the cross-section follows a plane that is substantially perpendicular to a main direction defined by the cavities.

Said cavity sections each having a surface area and a shape that are substantially equal throughout the entire implant, for instance if the section is a given polygon, then the same type of polygon is retained for the entire implant. For example, the cavity sections have a circular or elliptical shape. The external surface of the porous portion is a surface that defines the contour of the porous portion and that substantially does not reveal the openings of the cavities. Such an external surface of the porous portion is relatively non-substantive and comprises for example the ends of the walls delimiting the cavities.

Preferably, the cavities are connected with one another, or interconnected. Preferably, the cavities are connected to one another in a tangential manner, with substantially equal cavity sections. Preferably, the cavity sections are equal throughout the porous portion. For example, the cavity sections are equal throughout the dense portion.

Preferably, the cavities of the implant are channels.

A preferred embodiment of the implant comprises cavities or curved channels connected to one another. Such curved channels provide a controlled porosity in the implant, and in particular in the porous portion thereof. The curved channels are preferably obtained by the regular repetition of a pattern comprising concave curved channels. Such curved channels enable optimised cell proliferation.

A pattern, which can also be called cellular, features a biomimetic approach based on structures that have a TPMS (Triply Periodic Minimal Surface). In the context of the present invention, the cells and the patterns are defined in a three-dimensional space.

Such TPMS structures provide a high proportion of curved channels for a minimum average material density of the porous portion, for example between 30 % and 70%, more preferably between 40% and 60%.

Preferably, the curved channels are defined by a radius of curvature of curved channels. Such a radius of curvature of curved channels is between 300 µm and 1000 µm. A porous portion comprising curved channels defined by such radiuses of curvature enables a vast improvement of cell proliferation, of tissue integration and of vascularisation with respect to a porous portion that does not comprise such curved channels.

The advantage of an implant with a macroporosity in which the diameter of the channels and the distribution of the channels are well controlled is that it enables osseointegration and osteoconduction, and possibly osteoinduction and even bioresorbtion of the implant that are faster and more homogeneous. In this case, the term "channels" describes without distinction the cavities, the pores (of the macroporosity) or the channels. Furthermore, this allows the proper integration of the implant by the bone with which the implant is positioned to be in contact. This also achieves good mechanical properties.

The calcium and phosphate-based synthetic ceramic enables a reduction of implant rejection risks that is more satisfactory when using an implant according to the invention. For example, the invention provides better results than implants with a metal base, a polymer base, or with a base made of composite materials of the ceramic/polymer type.

The implant according to the invention having a macroporosity featuring curved channels yields results that are very similar to, and even better than the results achieved with autotransplantation. The implant according to the invention is an excellent compromise in terms of the mechanical properties, rejection risks, osseointegration, osteoconduction, osteoinduction and bioresorbtion, with respect to implants according to the state of the art.

Preferably, the implant according to the invention is designed to comprise an essentially dense portion or a dense portion or a portion featuring an elevated material density in the parts that would come into contact with the dura mater or with a membrane. However, the implant is designed to feature a maximum porosity at the points of contact with a bone or with cartilage. Such a distribution of the macroporosity based on the anatomic nature of the part with which the implant comes into contact improves the regeneration of the implant, i.e. a high bioresorbability on the bone or cartilage, thereby enabling the hardening/stiffening of the bone or cartilage at the point of contact.

Preferably, the implant according to the invention is an implant selected from the following types of implant: jaw filling implant, mandibular filling implant, implant used for the sealing of the cranium, implant for filling or repairing a facial bone, for example a cheekbone, nasal implant.

In addition to the biocompatible, and potentially bioresorbable properties of the materials used in the manufacturing of the implant, the invention provides an implant that provides good osseointegration properties. The macroporosity can enable either regrowth of the tissues in the cavities that characterise the macroporosity, or osteoinduction of the bone in the cavities. The macroporosity also provides a lighter implant, while retaining its shape and stiffness compared with an implant with the same aspect but without macroporosity.

The term "macroporosity" is used to describe a porosity with a minimum dimension that is preferably greater than 100 µm, and even more preferably greater than 200 µm. The macroporosity can be defined as a material density that corresponds to the ratio of the volume of the pores to the overall volume of the object. The pores are also cavities, holes or orifices.

The advantage of being able to vary the average macroporosity of the implant is to obtain an implant that has a variable macroporosity volume, a variable macroporosity diameter or a variable number of pores.

The term "average macroporosity of the implant" means that an average value of the macroporosity is defined for the entire facial implant. Similarly, it is also possible to use the term "average macroporosity of the porous portion". The average macroporosity corresponds for example to the integration of the macroporosity defined by the walls of the cavities of the porous portion with respect to the total volume of the porous portion. For example, several porous portions can have different macroporosity values; an average macroporosity value is a weighted average of the macroporosities of each porous portion.

The cavities are defined by side walls, the cavities are defined by sections oriented perpendicularly to the side walls. For example, in the case of a cylindrical cavity, the section corresponds to the diameter of a circle perpendicular to the tangent of the side wall. Preferably, in the case of a non-cylindrical cavity, the section is defined by a circle. The circle defining the section has a diameter encompassing the section, and the diameter of the circle defining a cavity section is preferably between 0.3 mm and 1.2 mm. Preferably, the cavities are not parallel with one another. For example, the cavities have sections with variable diameters. For example, a cavity comprises several sections with diameters ranging from 0.3 mm to 1.2 mm.

The invention has a macroporosity that enables, compared with a microporosity, a good population of the implant according to the invention by cells (bone or tissue) of the host body, enabling a proper integration of the implant by the host body, for example by osseointegration or by bioresorbability.

Implants according to the state of the art are cold manufactured in order the preserve the fragile calcium phosphate-based foam with which they are made. Such a foam being highly porous, it is therefore a fragile material. To overcome this fragility, such implants have to be thicker than the anatomical bone. This type of implant features good bone integration properties, but requires more voluminous implants with mechanical properties that are reduced with respect to the implants according to the invention.

A biocompatible ceramic of synthetic origin is for example manufactured with chemical synthesis methods.

The invention significantly reduces the contamination risks with respect to the use of an implant of animal origin or an implant of human origin coming from another donor.

The advantage of the invention with respect to a kit or cut prosthesis is to have a custom-made prosthesis that requires no adapting when it is being implanted. The invention thus enables a quick transplantation and having a prosthesis adapted to the patient.

A prosthesis or an implant adapted to the patient is a customisable implant that fits with the anatomical shapes of the patient, and that defines a chosen form of the bone to be repaired. Such a customisable implant has the advantage of being able to perfectly fill a bone defect. Such an implant can be better attached to the patient, by increasing the surface area of contact with the tissue and/or bone, thanks to the control of macro porosities. The advantage of the implant according to the invention is that it provides an implant featuring controlled macroporosities with well-defined positions that are preferably adjacent to the external surface of the implant.

The cavities are preferably pores that constitute the macroporosity. For example, the implant comprises microporosity in addition to the macroporosity.

Bioceramics are ceramics used as biomaterials for the manufacturing of implants or prostheses, for example as defined in the reference work, Biomechanics and Biomaterials in Orthopedics, T. Yamamuro. Bioceramics of synthetic origin are for example obtained by chemical or physical methods. Synthetic bioceramics are preferably made of synthetic materials. For example, synthetic bioceramics are obtained by chemical synthesis processes. For example, synthetic bioceramics are obtained by physical synthesis processes. For example, synthetic bioceramics are obtained by physical and chemical synthesis processes. For example, bioceramics of synthetic origin are made directly from materials of animal origin or from plant-based materials. For example, bioceramics of synthetic origin comprise raw materials and natural and synthetic additives that are implemented through industrial processes.

The advantage of the implant according to the invention with respect to an implant manufactured by injection, as described above, is that it enables a properly-controlled osseointegration and avoids having porous sections that are too far from an area of osseointegration. The advantage of having an implant according to the invention with respect to an implant comprising non-bioresorbable materials is that the implant according to the invention enables, over a relatively long period of time, a full bioresorbability of the implant, in order to leave space for the bone. This is particularly advantageous for implants intended for individuals who are not yet fully grown; indeed, the implant according to the invention has the advantage of being able to follow the growth of an individual, which a large majority of implants according to the state of the art do not allow.

Preferably, the porous portion is defined by the repetition of a first basic pattern, preferably in three dimensions.

Preferably, the first basic pattern is of the triply periodic minimal surface type; for example, it is of the gyroid type.

Preferably, the side walls delimiting at least partially the cavities are defined by a triply periodic minimal surface, preferably of the gyroid type. The advantage of such side walls is to enable improved tissue integration and bioresorbtion of the implant, thanks to the smooth wall providing the cellular, vascular and osteoinductor elements with a channelled slide covering several millimetres and centimetres.

Cavities delimited by a structure having a triply periodic minimal surface are preferably defined by a structure selected from the following structure types: gyroid; Schwarz P; Diamond; Neovius; iWP, Schwarz D, P W Hybrid; Holes; Icosahedron; L; Schwarz G.

Preferably, said side walls are smooth.

Preferably, said smooth side walls are defined by a mathematical function in three dimensions so that they are free of self-intersections.

Preferably, said side walls comprise a single non-intersected surface. The advantage of having a single surface that does not intersect with another surface is to achieve a surface that is guaranteed not to feature an abrupt change of curvature. An abrupt change of curvature is not recommended as it is not conducive to cell proliferation.

The advantage of having a triply periodic minimal surface is to achieve an interconnection of all the cavities without the walls of these cavities being connected, with forming intersections between their respective surfaces.

The term "smooth" means that the surface is not intersected by another surface, or that it is not self-intersected. Considering the manufacturing method of the implant according to the invention by additive manufacturing, there are steps or slight changes of surface orientation between two adjacent layers. The surface is slightly rough (0.1 to 10 µm), providing for improved anchoring of the cells to the surface, and improved interactions with the receptors of the cell membranes. Thus, the term "surface" is used to describe the surface that one has sought to create by additive manufacturing, said surface corresponding substantially to a shell that fits with the various layers of matter that are to be taken into account. The advantages provided by the side walls of the cavities are to be associated, in terms of their geometry, with the shell/contour thereof and not directly with the side walls as manufactured by additive manufacturing.

Preferably, the cavities are curved, and even more preferably, the curved cavities are defined by a radius of curvature ranging from 300 µm to 1000 µm, and yet even more preferably by a radius of curvature ranging from 500 µm to 750 µm.

The radius of curvature of a cavity is defined with respect to a line that is equidistant to the side walls of said cavity. The equidistance line intersects with the centre of each cavity section, the cavity sections being defined along a plane that is substantially perpendicular to a main direction defined by the cavity. In the case of curved cavities or cavities delimited by a triply periodic minimal surface, the main direction defined by the cavity varies in a continuous manner. For example, all the cavities of the porous portion have the same radius of curvature.

Preferably, the porous portion has a gradient of material density by volume between the connection zone and the external surface such that the material density of the porous portion is greater at the level of the connection zone than it is at the level of the external surface.

The advantage of having such a porosity gradient is to achieve good mechanical properties of the porous portion and, in particular, said porosity gradient provides a porosity directly under the external surface that has reduced material density, ranging from 30% to 40%, but that is sufficiently robust as is it supported by a macroporosity that has greater material density. Thus, such a macroporosity gradient of the porous portion provides osseointegration properties that are as good as a porous portion having the same material density throughout its entire thickness. The implant according to the invention, featuring a porosity gradient, provides good osseointegration properties, firstly at the level of the porous portion directly in contact with the bone, while also having good mechanical properties of the porous portion. Indeed, certain implants with a constant porosity throughout the implant either feature good osseointegration but relatively poor mechanical properties, for instance in the direct vicinity of the osseointegrated part, or feature poor osseointegration properties but good mechanical properties of the implant. Thus, the implant according to the invention, with a macroporosity featuring a gradient of material density, provides a good compromise between the mechanical properties of the implant and the osseointegration properties of the implant. The implant thereby reduces the convalescence or immobilisation time after the transplantation of the implant, and achieves better results.

Preferably, such a porosity gradient can be defined by a variation of material density per unit of distance. Thus, the porosity gradient can be defined by a variation of the material density of the porous portion. For example, a variation of the porosity occurs along a rectilinear direction substantially perpendicular to the external surface of the porous portion or to the connection zone with the dense portion. Such a porosity gradient of the porous portion along the direction defined above can vary from 3% per mm to 1 % per µm, for example from 10% per mm to 10% per µm, for example from 5% per mm to 15% per mm. Preferably, such a porosity gradient expressed in this manner is variable within the same porous portion of the implant. Indeed, depending on the thickness of the porous portion, the gradient per unit of length will vary to achieve a relatively low material density on the outside of the porous portion, and a material density close to 100% at the level of the connection zone.

Preferably, the material density gradient is substantially defined by values ranging from 30% to 40% at the level of the external surface and from 70% to 100% at the level of the connection zone.

The advantage of such a distribution of the material density is to achieve a porosity with cavities or pores with large openings and walls between the cavities that are relatively thin with respect to the size of the openings. With this type of porosity in contact with the bone, it is possible to achieve quick osteoinduction inside the pores, leading to optimally efficient osseointegration in terms of the speed and solidity of the anchoring of the implant to the bone. A more reduced porosity, i.e. a greater material density, in the vicinity of the dense portion, provides good mechanical properties while ensuring an osseointegration in the vicinity thereof, until it reaches said dense portion. This also enables better adaption to the osteoinduction kinetics, which will be faster in the immediate vicinity of the bone than at a greater distance from the bone, i.e. at the level of the dense portion.

The material density should be determined by considering a volume comprising a plurality of cavities as well as a plurality of walls of said cavities. Preferably, the material density of the porous portion will be determined by considering a volume comprising at least one pattern that is repeated, for example at least twice, for example at least ten times, for example at least one hundred times, substantially around said at least one pattern. Preferably, such a measurement of material density is performed in a volume between the external surface of the porous portion and a first imaginary internal surface parallel with the surface of the porous portion. The portion of the external surface and the first imaginary internal portion are positioned with respect to one another so that a line perpendicular to the external surface is perpendicular to the first imaginary internal surface at substantially every point of each of these surfaces. The volume defined by the space between these surfaces enables to determine a material density at the level of the external surface of the porous portion.

The material density of the porous portion at the level of the connection zone or at the level of the dense portion, i.e. in the immediate vicinity of the connection zone or of the dense portion, can be determined in a similar manner as for the material density of the porous portion at the level of the external surface. Thus, the material density should be determined by considering a volume comprising a plurality of cavities as well as a plurality of walls of said cavities. The material density of the porous portion at the level of the dense portion can comprise cavities that are sealed by the dense portion. Preferably, the material density of the porous portion will be determined by considering a volume comprising at least one pattern that is repeated, for example at least twice, for example at least ten times, for example at least one hundred times, substantially around said at least one pattern. Preferably, such a measurement of material density is performed in a volume between the connection zone of the porous portion, and a second imaginary internal surface parallel with the surface of the porous portion. The connection zone and the second imaginary internal surface are positioned with respect to one another so that a line perpendicular to the external surface is perpendicular to the second imaginary internal surface at substantially every point of each of these surfaces. The volume defined by the space between these surfaces enables to determine a material density at the level of the connection zone of the porous portion with the dense portion. Preferably, the distance from the external surface and the first imaginary internal surface along the line drawn perpendicularly thereto ranges from 1 µm to 5 mm, for example from 10 µm to 2 mm, for example from 50 µm to 1 mm, and is for example 100 µm or 50 µm.

Preferably, the gradient of material density is substantially defined between said connection zone and the walls adjacent to the external surface delimiting said opening of the porous portion.

Preferably, a material density is measured so as to achieve measurement that is representative of the material density of the porous portion. The material density is measured in a volume of the concerned portion that is representative and provides a measurement that can be reproduced, regardless of the location of the measured zone.

Preferably, the cavities of the porous portion are interconnected, and more preferably they are all interconnected.

Preferably, the cavities open onto the external surface through openings.

Preferably, the diameters of the cavities are distributed according to at least one gradient substantially defined between the connection zone and the openings of the porous portion.

For example, the diameters of the cavities at the level of their openings range from 1.2 mm to 500 µm, preferably from 1.1 mm to 600 µm, and even more preferably, from 1 mm to 700 µm. For example, the diameters of the cavities at the level of the connection zone, in the immediate vicinity thereof, for example at the level of the second imaginary surface located at 500 µm or at 1 mm of the connection zone, range from 500 µm to 0, preferably from 400 µm to 0, and even more preferably from 300 µm to 0.

Preferably, the diameters of the cavities are greater at the level of said openings and smaller at the level of the connection zone.

For example, the diameters of said cavities are substantially twice the size at the level of the openings than they are at the level of said connection zone, preferably three times the size, and even more preferably, four times the size.

Preferably, the implant is a cranial-maxillofacial implant. For example, the implant is intended for the cranium, for example, the implant is intended for an upper jaw bone, for example, the implant is intended for a face bone.

With regard to the use of implants in facial repair surgery, the state of the art proposes the two following alternatives: 1. using a synthetic implant that can cause a reaction to a foreign body, 2. sampling a healthy bone, creating additional trauma in an area of the body where there was none before. The decision often stems from the experience of the surgeon or the patient. The present invention provides a synthetic implant that does not cause a reaction to a foreign body and that has biological properties that are similar to those of a graft. Furthermore, the implant according to the invention provides mechanical properties adapted to the purpose of the implant. Thus, the implant is particularly innovative as it proposes a custom-made synthetic implant that causes no rejection reaction, enabling good osseointegration, and that is optimised for its mechanical properties. To achieve this, the implant is custom-made by 3D printing with a synthetic bone composition (hydroxyapatite and tricalcium phosphate) with controlled porosities and densities in order to combine biological and mechanical properties.

Preferably, a cranial implant is used to reconstruct a cranial hollowness with a bone substitute. The problem with the state of the art is that there is no bone substitute that combines adaptability features in the operating theatre with osseointegration and resistance to shocks that are similar to those afforded by real bone. The implant according to the invention provides a cranial implant that is resistant, adaptable, biocompatible, and osseointegrable. Hydroxyapatite is the preferred material to enable the bone to integrate inside the implant and, with time, to transform the implant into bone material. With the implant according to the invention, cranioplasty, in addition to being reconstructive surgery, can also be regenerative surgery.

To overcome the main problems created by the brittle nature of hydroxyapatite, the impossibility of adapting it in the operating theatre or of creating thin structures, and the extended manufacturing time of the implant with standard machining techniques (approximately six weeks), it is possible, depending on the required application, to consolidate the implant according to the invention with PEEK or PMMA. These materials, largely used in neurosurgery, have mechanical properties that are close to that of bone, while also being adaptable in the operating theatre. Furthermore, thin structures can be reproduced with PEEK/PMMA, which is not the case for hydroxyapatite implants. Based on the post-treatment carried out on hydroxyapatite, a peripheral adaptability is also possible. An implant made both of hydroxyapatite and of PMMA/PEEK enables to solve issues inherent to cranioplasty by combining osseointegration, adaptability and solidity.

Preferably, the cavities are sealed at the level of the connection zone.

The cavities are sealed at the level of the connection zone, between the dense portion and the porous portion. When the cavities are sealed, a part of the porous portion in question is considered as a part of the dense portion.

Preferably, said porous portion is a macroporous section.

The porous section comprises primarily macro cavities, i.e. with dimensions featuring a diameter substantially defined between 100 µm and 1 mm. The presence of micropores is not excluded.

Preferably, the macroporosity is an open porosity.

An open porosity means that all the cavities defining the macroporosity open onto the external surface, i.e. there is a communication towards the external surface for each of the cavities. The open macroporosity means that the osteoinduction when the porous portion is positioned to be in contact with a bone can occur in all the cavities. This is particularly advantageous as it helps prevent potentially infectious zones from being present in the implant, for example when the cavities are sealed or don't communicate with the external surface. Indeed, such closed cavities, during the bioresorbtion of the implant, could cause infections.

Preferably, said dense portion is defined by the repetition of a second basic pattern, preferably in three dimensions. For example, the second basic pattern is of the gyroid type. The various versions, properties and characteristics of the first basic pattern apply to the second basic pattern, *mutatis mutandis.*

Preferably, the implant comprises at least an attachment hole through said dense portion and/or said porous portion.

An attachment hole enables for example the passage of a means to attach the implant to the bone, to cartilage, to a membrane or to tissue, the attachment means being for example an attachment screw or an attachment stitch. The passage of such an attachment means enables proper positioning of the implant at the moment of its implantation and during the postoperative phase. In particular, the advantage of having a hole for attachment purposes that is performed through the dense portion and/or the porous portion is that it enables potentially applying an effort with the attachment means such that the porous portion of the implant is maintained in contact with the bone requiring said implant. Preferably, the attachment hole is performed only through the dense portion, i.e. there is no porous portion on the part of the implant that comprises said attachment hole. Thus, an implant attached through an attachment hole performed only through the dense portion can be attached with a relatively strong support at the contact point, as the attachment means in contact with the dense portion provides for a greater tightening torque of a screw than when the attachment means is in contact with a porous portion. Indeed, when the attachment means is in contact with a porous portion, there is a risk of damaging the porous portion. For example, the attachment hole is performed through a dense portion and through a porous portion. The dense portion is located on the side of the hole intended to receive the head of the attachment screw, i.e. intended to be in a state of constraint caused by the screw head, and the porous portion is located on the other side of the attachment screw, i.e. intended to be in contact with the bone.

Preferably, the implant comprises at least two dense portions, said implant comprising at least two attachment holes performed through said at least two dense portions.

Preferably, the implant further comprises:
- an intermediate portion connected to said dense portion and to said porous portion, and featuring an average macroporosity characterised by:
   ∘ an average material density determined for the entire volume of said intermediate portion of between 50 % and 90 % by volume;
   ∘ cavities delimited at least partially by side walls defining cavity sections, said cavity sections each having an extension such that it is possible to fit each of said cavity sections of the intermediate portion in a circle having a diameter ranging from 0.2 mm to 0.7 mm;
   said dense portion, said intermediate portion, and said porous portion define at least partially an external surface of the implant;
   said cavities open onto said external surface.

Preferably, the intermediate portion is defined by the repetition of a third basic pattern. The different versions, properties and characteristics of the first basic pattern apply to the third basic pattern *mutatis mutandis.*

Preferably, said at least two dense portions are attached to one another by means of said intermediate portion.

Preferably, the cavities of the porous portion and the cavities of the intermediate portion are interconnected, preferably all the cavities are interconnected.

Preferably, the dense portion, when it features a material density by volume ranging from 70% to 99%, comprises cavities delimited at least partially by side walls defining cavity sections of the dense portion, said cavity sections of the dense portion each having an extension such that it is possible to fit each of said cavity sections of the dense portion in a circle having a diameter ranging from 0.05 mm to 0.2 mm;

Preferably, the dense portion and/or the porous portion comprise a periodic basic pattern in a three-dimensional space, said basic pattern defining:
dimensions of the basic pattern in three dimensions,
thicknesses of the side walls ranging from 0.05 mm to 1.2 mm,
cavity sections ranging from 0.05 mm to 1.2 mm.

The advantage of such a basic pattern for the formation of a portion of the implant is that it enables changing the volume density of a portion of the implant, by modifying, for example, the thickness of the side walls for a same dimension of the basic pattern in three dimensions. Another advantage of having such a network of basic patterns for the creation of a portion of the implant is that it enables changing the number of cavities per unit of volume, by varying the dimensions of the implant in three dimensions, while retaining the volume density of the portion of the implant. Preferably, a portion of the implant formed by the network of basic patterns according to the invention comprises smooth side walls. Such smooth walls work much in the same way as a "slide", i.e. without an abrupt change of the slope of a tangent moving between two points located close to one another, for example at a distance of 0.5 mm or 1 mm from one another.

Preferably, the basic pattern can fit fully inside a cube with sides that range from 1 mm to 10 mm, preferably from 3 mm to 7 mm.

Preferably, the cavities of the porous and dense portions substantially communicate with one another. As mentioned above, the use of a gyroid-type basic pattern provides for a good interconnection of the porosities between portions with different densities, by changing only the thickness of the side walls delimiting the cavities. For example, such basic patterns provide an implant comprising porous portions and dense portions with a connection of all the porosities. For example, such gyroid-type basic patterns achieve the connection of all the porosities by varying the thicknesses of the side walls of a porous portion, of an intermediate portion and of a dense portion.

Preferably, the pattern density ranges from 1 to 1000 per cm³, preferably from 20 to 500 per cm³. Depending on the pattern density, it is possible to vary a porosity/cavity density per unit of volume, which is particularly advantageous for the implant according to the invention.

Preferably, the dense portion and the porous portion comprise a basic pattern that has substantially the same cavity positions; preferably the basic pattern is identical.

As explained above, the use of a same basic pattern enables an excellent interconnection of the cavities and the side wall, which is conducive to good osteoconduction.

Preferably, the side walls of the dense portion have thicknesses of the side walls ranging from 0.5 mm to 1.2 mm, and said side walls of the porous portion have thicknesses of the side walls ranging from 0.05 mm to 0.5 mm.

The advantage of causing the wall thicknesses to vary is to enable faster or slower bioresorbtion based on the thickness of the side walls.

Preferably, said side walls of the intermediate portion have thicknesses of the side walls ranging from 0.3 mm to 1.0 mm.

Preferably, said at least one attachment hole has a diameter of at least 1 mm, preferably ranging from 1 mm to 3 mm, for example 1 mm, 1.25 mm, 1.5 mm, 1.75 mm, 2 mm, 2.5 mm.

Preferably, said at least one hole is present at the level of a portion of the implant that has a minimum dimension of at least 2 mm, preferably at least 3 mm and even more preferably at least 5 mm.

A minimum dimension means that, at the level of the attachment hole, a minimum dimension is measured between the contours of the implant, excluding the attachment hole. For example, the contours of the implant are defined by the external surface of the implant; i.e. the external surface of the dense portion and/or of the porous portion.

Preferably, the at least one attachment hole has a main direction that is substantially parallel with a direction defined by the minimum dimension.

This means that the hole has a direction that enables the passage of an attachment means located in the smallest thickness of a portion intended to receive an attachment hole.

Preferably, the diameters of the cavities are distributed periodically in the implant.

The advantage of having periodically distributed cavity diameters is that they enable achieving a well-defined macroporosity in the porous portion, and achieving the most homogeneous osseointegration possible, along with the most homogeneous mechanical properties possible in the porous portion.

Preferably, said cavities of said porous portion are arranged according to a meshing of the cavities so that substantially all the cavities can communicate with one another.

Thus, all the cavities are said to be open, either because they communicate directly with the external surface, or because they communicate indirectly through other cavities by an interconnection system of the cavities towards the external surface.

Preferably, the cavities all have a communication with the external surface.

Preferably, the implant comprises at least two separate porous portions, said at least two porous portions being attached to said dense portion by said connection zone.

Several porous portions can be arranged around or in contact with a same dense portion. For example, an implant can have two porous portions intended to enable mechanical anchoring by osseointegration with a bone on several parts of the bone, or to several bones, or to several parts of a same bone.

The invention provides an implant made of elements that are naturally present in the human body, such as calcium phosphates or for example hydroxyapatite and tricalcium phosphate.

Preferably, said biocompatible ceramic comprises a synthetic bioceramic.

The bioceramics are biocompatible. Preferably, the bioceramics are bioresorbable.

Preferably, said biocompatible ceramic comprises a bioglass.

Preferably, the biocompatible ceramic comprises a bioceramic composition ranging from 0 to 100% and a bioglass composition ranging from 0 to 100%.

Preferably, the biocompatible ceramic comprises a bioglass. Bioglasses are biocompatible. Preferably, the bioglasses are bioresorbable.

Preferably, the biocompatible ceramic comprises a bioglass composition ranging from 0 to 100%. Preferably, the bioglass being used is made primarily of silicon oxides, sodium oxides, calcium oxides and phosphorous oxides. Preferably, the bioglass is used as a replacement of tricalcium phosphate. Preferably, the bioglass can resorb itself chemically. For example, the bioglass being used has a percentage by weight of 45% of silicon oxides, 24.5% of sodium oxides, 24.5% of calcium oxides and 6% of phosphorous oxides.

The advantage of using bioglass resides in its biological activity in the presence of living bone. In the presence of living bone, bioglass enables quicker regrowth of the bone in the part that is exposed to the bioglass. For example, the bioglass is slightly more biologically active than tricalcium phosphate, and is sufficiently compact.

Preferably, the biocompatible ceramic comprises a calcium phosphate composition ranging from 0 to 100%.

A composition of calcium phosphate preferably has the following: hydroxyapatite (HAP) and tricalcium phosphate (TCP). These are therefore biocompatible and bioactive materials, i.e. they react positively with the organism, by having a chemical activity with the physiological environment and by creating biological bonds with the bone tissue, thereby enabling osseointegration in order to transmit the shear forces from the bone to the implant and *vice versa.*

Preferably, the biocompatible ceramic comprises a hydroxyapatite composition ranging from 0 to 100%, and more preferably ranging from 50% to 100%, and even more preferably ranging from 55% to 97%. Other composition examples are as follows: between 20% and 100%, between 0 and 80%, between 40% and 90% and between 10% and 60%.

Preferably, the compositions of the facial implant are given in percentage values with respect to the weight of each component. A hydroxyapatite composition of the facial implant ranging from 0 to 100% indicates that the facial implant comprises from 0 to 100% of hydroxyapatite.

The benefit of using hydroxyapatite is to take advantage of its bioactive nature in order to enable improved tissue regrowth around and inside the implant. The benefit of using calcium phosphates as, for example, tricalcium phosphate, is to take advantage of its biodegradable and bioresorbable nature. The advantage of using a composition comprising hydroxyapatite and tricalcium phosphate is to achieve a material combining biological activity and bioresorbability properties.

Preferably, the biocompatible ceramic comprises a tricalcium phosphate composition ranging from 0 to 100%, and more preferably ranging from 0 to 50%, and even more preferably ranging from 3% to 45%. Other composition examples are as follows: between 0 and 90%, and between 55% and 75%.

The hydroxyapatite and tricalcium phosphate composition of the implant is conducive to good biocompatibility of the implant with the body of a host. Hydroxyapatite and tricalcium phosphate are naturally present in the human body, and more particularly in the bony matrix of the bones. This composition is also conducive to bioresorbability as these compounds are naturally soluble in water, and therefore in the human body.

The advantage of being able to vary the hydroxyapatite composition from 0 to 100% and the tricalcium phosphate composition from 0 to 100% is that it enables changing the bioresorbability speed. Indeed, tricalcium phosphate is more soluble in water than hydroxyapatite and therefore degrades faster in the human body. An implant with a TCP composition that is close to 100% features relatively fast bioresorbability compared with an implant of which the composition is for example close to 60% of hydroxyapatite and 40% of tricalcium phosphate.

Preferably, the biocompatible ceramic further comprises a dicalcium phosphate composition ranging from 0 to 100%.

Preferably, the biocompatible ceramic further comprises an amorphous calcium phosphate composition ranging from 0 to 100%.

Preferably, the biocompatible ceramic further comprises a tetracalcium phosphate monoxide composition ranging from 0 to 100%.

Preferably, the tricalcium phosphate in an alpha form.

Preferably, the tricalcium phosphate in a beta form.

Preferably, the tricalcium phosphate in a gamma form.

The nasal implant can also be made of other forms of calcium phosphate in all proportions, i.e. amorphous calcium phosphate (ACP), dicalcium phosphate (DCP), alpha tricalcium phosphate (α-TCP), beta tricalcium phosphate (β-TCP), gamma tricalcium phosphate (γ-TCP), hydroxyapatite (HAp),and tetracalcium phosphate monoxide (TCP).

Preferably, the implant can also comprise compounds such as calcium sulphates, calcium carbonates, and for example hydroxycarbonate apatite or apatite-wollastonite.

The advantage of using one or several of these materials is that it makes it possible to adapt the composition of the facial implant to the required mechanical properties and bioresorbability properties. The advantage of using calcium phosphates with different stoichiometry values and crystal structures is that it enables modifying the solubility of the implant in water, for example. The advantage of using calcium phosphates with different stoichiometry values and crystal structures is that it enables modifying the bioresorbtion speed.

The hydroxyapatite is preferably pentacalcium hydroxide tris(orthophosphate) (HAp).

Preferably, the biocompatible ceramic comprises a bioglass composition ranging from 0 to 100%.

Preferably, the cavity sections are polygonal.

Preferably, the cavities of the porous portion are substantially parallel with one another, for example they are parallel with one another.

Preferably, said cavity sections all have a surface area and a shape that are substantially equal throughout the entire implant.

Preferably, the diameters of the cavities are distributed homogeneously throughout the implant.

Preferably, the external surface is covered with a coating.

Preferably, said coating is made of a biocompatible material.

The implant is preferably manufactured by 3D printing. The facial implant 5 is for example manufactured by additive layer manufacturing (ALM).

According to a second aspect of the invention, the inventors propose a method for manufacturing an implant according to the first aspect of the invention, comprising the following steps:
a. defining a design space of the implant, said design space being defined by a space that requires filling by the implant;
b. defining, for said implant, the following design parameters:
   - the required mechanical properties of the implant;
   - the positions of the external surfaces of the implant;
   - a nature of the tissues and/or materials delimiting the design space of the implant;
c. defining an implant that substantially fits with the design space, said implant comprising at least one dense portion such as defined according to any one of the preceding claims, and at least one porous portion such as defined according to any one of the preceding claims based on the nature of the tissues and/or materials delimiting the design space;
d. optimising a shape of said implant within said design space by using a topology optimisation method taking into account said design parameters of said implant, so as to achieve an optimised shape of said implant;
e. providing an additive manufacturing machine;
f. providing a program for the breakdown into slices defining a number M of layers and the geometry of the layers that are to be deposited for the additive manufacturing of said implant, M being a positive integer equal to or greater than 2;
g. providing a material to be deposited that comprises synthetic bioceramic;
h. depositing a layer of said material to be deposited on a support;
i. solidifying said layer;
j. repeating said steps d. and e. M - 1 times, such as defined by said breakdown program;
k. removing said material to be deposited that has not been solidified;
l. conducting a heat treatment of said printed layers in order to consolidate the remaining fraction.

The different versions and advantages of the implant according to the first aspect apply to the process according to the second aspect of the invention, *mutatis mutandis.* In particular, the different versions of the suggested patterns according to the first aspect are applicable to the optimisation step d. The step d. preferably comprises a sub-step whereby a basic pattern is selected from the patterns described for the first aspect of the invention.

The advantage of using a method relying on an additive manufacturing machine for the manufacturing of an implant is that it makes it possible to manufacture a custom-made implant. The dimensions of the implant can be modified just before the manufacturing of the implant, to ensure that the implant meets the patient's or operating surgeon's expectations. Thus, this manufacturing technique does not require a mould or sacrificial material, for example in polymer foam placed in a mould and in which/around which a ceramic-based material is hardened and sintered to provide a porous ceramic material.

Another advantage of using such an additive manufacturing process (3D printing) is that it enables manufacturing porosities (channels) featuring a geometry optimised for cell proliferation, tissue integration and vascularisation. This optimised geometry of the porosity (channels) is described for the implant according to the first aspect of the invention.

Another advantage of 3D printing is that it enables manufacturing with a controlled material density and, in particular, with well-defined porous portions and dense portions. The dense portions provide good mechanical properties to the implant, and the porous portions provide good osseointegration properties and good bioresorbtion properties. Furthermore, this manufacturing technique enables the manufacturing of implants that have optimised mechanical properties and a position of the cavities or of the porosities that is well controlled, along with a thickness of the walls and a depth of the cavities that are also well controlled. This implant design featuring an interleaving of dense and porous structures is advantageously achieved by the method of additive manufacturing according to the second aspect of the invention. This interleaving makes it possible to combine osseointegration, osteoconduction, osteoinduction and mechanical properties of the implant.

The implementation of a method by 3D printing also provides an implant featuring interconnected macroporosity, which is conducive to the satisfactory cell regrowth of the implant. 3D printing makes it possible to change the composition of the material to be deposited, and therefore the implant, without having to make important changes to the 3D printer.

The 3D printing method enables manufacturing a custom-made implant, with a custom-designed composition and an interconnected macroporosity conducive to the satisfactory regrowth of tissue, and therefore a good support of the implant. The combination of the composition of the implant and of the macroporosity achieved by 3D printing provides an implant allowing fast osseointegration and/or a bioresorbable and/or a partially bioresorbable implant.

The method according to the invention makes it possible to design an implant with integration properties that are equal to or better than that achieved with a calcium phosphate foam and the correction of the defects inherent to such a foam, in particular in terms of the mechanical properties of such a foam. For this purpose, the method according to the invention makes it possible to determine a porosity that is sufficient to enable satisfactory integration, without it affecting the mechanical stability of the implant. The manufacturing method according to the invention combines topological simulation tools and 3D printing. Thus, it is possible to increase the density of the parts of the implant where constraints are exerted, and to keep the same porosity in the parts where the constraints are reduced. This type of design also takes into account the nature of the tissue and/or material delimiting the design space, in order to ensure optimal integration of the implant.

For example, the implant is manufactured by means of an additive manufacturing method such as 3D printing.

Preferably, the material that is to be deposited comprises synthetic bioceramic and additives (polymers, photoinitiators, resins, glues).

Preferably, the manufacturing method comprises the following additional step:
- defining the location of at least one attachment means, preferably an attachment hole, based on the nature of the tissue and/or materials delimiting the design space of the implant.

Preferably, the shape of the implant comprises a periodic repetition of a basic pattern.

Preferably, the basic pattern comprises cavities, delimited at least partially by cavity walls, said cavity walls having cavity thicknesses defined between two adjacent cavities, said cavity thicknesses ranging from 0.05 to 1.2 mm.

Preferably, the topology optimisation method makes it possible to vary the cavity thicknesses of said basic pattern so as to provide said optimised shape of said implant. The variation of the thicknesses of the side walls delimiting at least partially the cavities makes it possible, for a same dimension of the basic pattern, to achieve a variation of the material density of a portion of the implant. Thus, such a variation of the thickness of the side walls enables the formation of the porous, intermediate and dense portions.

For example, the additive manufacturing machine comprises a source of light and an optical projection system.

For example, the material to be deposited has a viscosity greater than 1 Pa.s.

The advantage of using a material to be deposited having high particle content is that it reduces to a minimum the shrinkage of the end part with respect to the solidified part. Shrinkage is in particular observed during the debinding and sintering steps. The advantage of using a printing material having high particle content is that it enables to obtain a dense facial implant. The advantage of using a printing material with high particle content is that it provides a facial implant with a well-controlled interconnected macroporosity.

### Brief description of the figures

These aspects, as well as other aspects of the invention, are clarified in the following detailed description of specific embodiments of the invention, with reference to the drawings of the figures, in which:
- Figs. 1a to 1c show schematic cross-section views of several embodiments of an implant according to the invention;
- Figs. 2a to 2c show schematic cross-section views of several embodiments of an implant according to the invention;
- Figs. 3a to 3c show schematic cross-section views of several embodiments of an implant according to the invention;
- Figs. 4a to 4c show schematic views of several embodiments of an implant according to the invention comprising attachment holes;
- Figs. 5a and 5b show schematic views of an embodiment of an implant according to the invention;
- Figs. 6a and 6b show views of a basic pattern; Fig. 6c shows a schematic view of a portion of an implant comprising 8 basic patterns;
- Fig. 7a shows a basic pattern and Fig. 7b shows an assembly of several basic patterns according to Fig. 7a.

The drawings of the figures are not to scale. Generally, similar elements are designated by similar references in the figures. The presence of reference numbers in the drawings cannot be considered as being limiting, including when these numbers are provided in the claims.

### Detailed description of certain embodiments of the invention

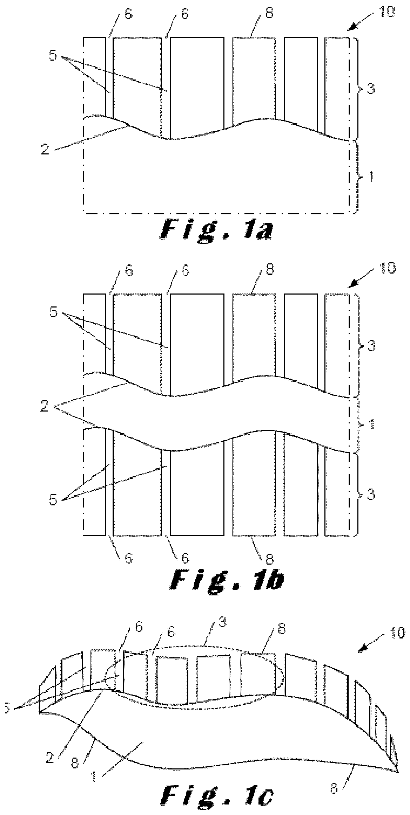

Figure 1a shows an example of an embodiment of an implant 10 comprising a dense portion 1 and a porous portion 3 delimited by a connection zone 2. The implant 10 has an external surface 8 that delimits the dense 1 and porous 3 portions from the exterior. The porous portion 3 comprises cavities 5 defining the macroporosity of the implant 10. These cavities 5 are schematically represented by parallel hollow sections and, when in the same plane, should be considered as a set of cavities in a three-dimensional space. The cavities 5 also comprise interconnections under the surface 8, so as to form a meshing of cavities. The cavities 5 communicate with the external surface 8 through openings 6. Figure 1a can be seen as an example of a cross-section view of an implant 10 according to the invention, not showing the interconnection between the cavities 5, although such an interconnection exists.

Figure 1b shows a different version of Fig. 1a where the dense portion 1 is central in the implant 10 and where the porous portion 3 is peripheral to the implant 10. The dense portion 1 therefore plays the role of a skeleton to provide the implant with good mechanical properties, and the peripheral porous portion enables good osseointegration during the implantation thereof. The porous portion 3 located in the periphery of the dense portion 1 can comprise several porous portions 3, for example there exists under the external surface 8 of the implant 10 an alternation of dense parts of the dense portion 1 and an alternation of porous portions 3.

Fig. 1c shows another version of Figs. 1a and 1b. The porous portion 3 in contact with the dense portion 1 at the level of the connection zone 2 defines a height of the porous portion that can be varied and fits with the surface of the dense portion 1. Fig. 1c shows an example of an implant for which, under the external surface 8, there exist zones of dense portion 1 and of porous portion 3. Furthermore, the porous portion 3 can have different heights depending on its location and possibly based on the bony part with which the implant is to be in contact. Furthermore, the walls of the cavities of the porous part can have different thicknesses based on various requirements in terms of osseointegration speed or of bioresorbtion speed.

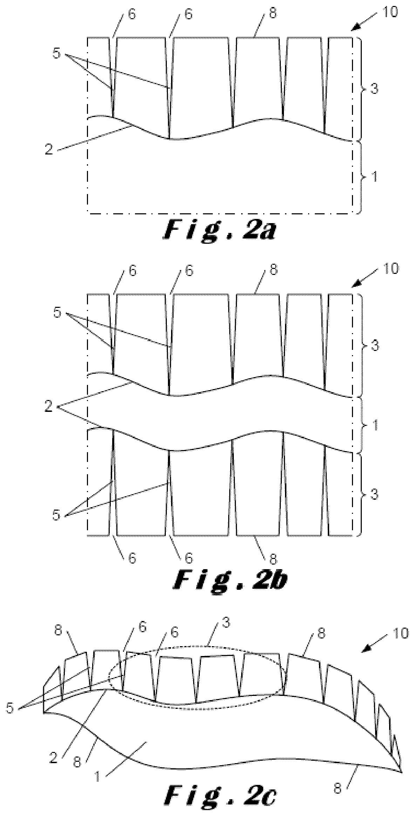

Fig. 2a shows an example of an embodiment of an implant 10 such as described in Fig. 1a and for which the material density of the porous portion 3 is distributed so that the material density is greater at the level or at the point of contact of the connection zone 2 than at the level of the external surface 8 of the porous portion 3. The distribution of the material density is schematically shown by the cavities 5 such as represented, with cavity diameters at the level of their communication 6 with the external surface 8 that are greater than the cavity diameters at the level of the connection zone 2. This implies, among other aspects, that the thickness of the walls of the cavities is greater at the level of the connection zone 2 than at the level directly underneath the surface 8. Based on this schematic representation, the implant can therefore feature very different external aspects, as is shown in Figs. 2b and 2c.

Fig. 2b shows an example of an implant such as described in Fig. 1b and comprising the material density distribution properties in the porous portion 3 with respect to the dense portion 1 such as described for Fig. 2a.

Fig. 2c shows an example of an implant such as described in Fig. 1c and comprising the material density distribution properties in the porous portion 3 with respect to the dense portion 1 such as described for Fig. 2a.

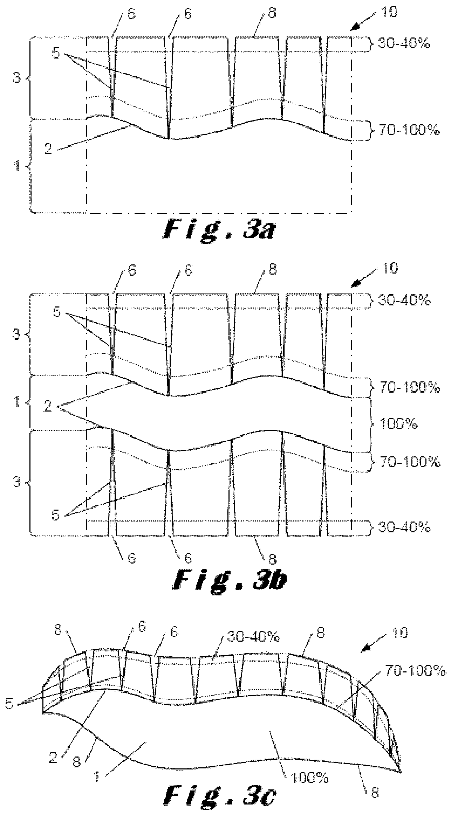

Fig. 3a shows an example of an implant 10 such as described in Fig. 2a, for which the porous portion 3 located under a large part of the external surface 8 delimiting the porous portion 3, i.e. over a thickness of 1 to 5 mm, has a material density ranging from 30% to 40%. The porous portion 3 located directly in contact with the dense portion 1 at the level of the connection zone has a material density ranging from 70% to 100%. The material density of the porous portion is always strictly inferior to 100%. The distribution of the material density between the two ends of the porous portion 3 can be linear, i.e. the material density distribution varies linearly with its position throughout the thickness of the porous portion 3. The distribution of the material density in the porous portion 3 can also be logarithmic, i.e. the distribution varies quickly in the vicinity of the external surface, and more slowly in the vicinity of the connection zone. The dense portion 1 has a material density equal to 100%, or in any case tending towards 100%. A dense portion in which the material density is of less than 100% may be considered if the manufacturing process does not achieve a material density of 100%. A dense portion 1 in which the material density is slightly less than 100% would not have a macroporosity such as that of the porous portion 3. A dense portion 1 in which the material density is slightly less than 100% would possibly have a closed porosity, contrary to the porosity of the porous portion 3, which is open.

Fig. 3b shows an example of an implant such as described in Fig. 2b and comprising the material density distribution properties in the porous portion 3 with respect to the dense portion 1 such as described for Fig. 3a.

Fig. 3c shows an example of an implant such as described in Fig. 2c and comprising the material density distribution properties in the porous portion 3 with respect to the dense portion 1 such as described for Fig. 3a.

In Figs. 1a to 3c, the external surface 8 covering the porous portion 3 is continuous, i.e. it comprises a surface in which the openings 6 of the cavities 5 create holes.

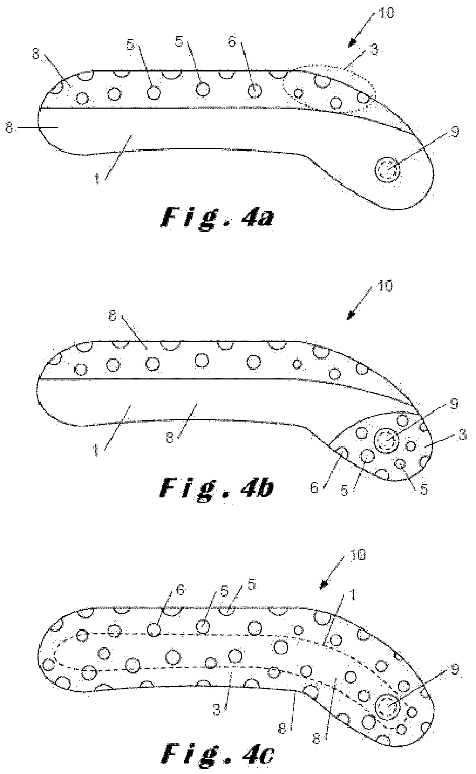

Fig. 4a shows an embodiment example of the implant according to any one of the embodiments listed in the description of Figs. 1a to 3c. The implant shown in Fig. 4a displays a porous portion 3 attached to the dense portion 1. The porous portion 3 has cavities 5 that open onto the external surface 8 by openings 6. The dense portion 1 features an attachment hole 9 to attach the implant 10 to a bone or to another part of the body on which the implant is intended to be implanted. Preferably, the implant 10 is mechanically coupled to a bone by an attachment means such as a screw that is screwed through the attachment hole 9. The screw has a screw diameter that is equal or inferior to the diameter of the attachment hole 9, and the screw head has a diameter that is greater than the diameter of the attachment hole 9. The location of the openings 6 of the cavities 5 is provided purely by way of example. The implant can have several attachment holes 9, for example two attachment holes 9 being positioned on either side of an implant with a substantially longitudinal shape. The attachment hole can, for example, include a location for the screw head that is recessed in the implant so that when the screw is in place to support the implant, the screw head is substantially below the external surface 8. For example, the attachment hole 9 features a geometry specific to a screw head, in particular to ensure that the forces applied by the screw during its tightening on the implant are correctly distributed throughout the implant. A large contact area between the screw head and the implant 10 is preferred.

Fig. 4b shows an example of an implant such as defined in Fig. 4a and comprising a porous portion 3 surrounding the attachment hole 9. For example, the porous portion is located at the level of a single face of the implant 10 featuring an opening of the attachment hole 9.

Fig. 4c shows an example of an implant such as described in Figs. 4b and 4a and comprising a porous portion 3 on the entire periphery of the implant, the dense portion being represented as a dotted line is located under the porous portion 3 and ensures that the implant 10 features sufficient mechanical properties.

The implant 10 is preferably made by 3D printing with a material featuring a very high particle concentration of materials enriched in hydroxyapatite and tricalcium phosphate. The implant 10 preferably undergoes chemical and thermal treatments to remove all organic components. Preferably, the particles of materials enriched in phosphate and calcium are sintered to obtain an implant 10 made of ceramic material.

The bioglass that can be used in the implant 10 has for example a composition by weight with 45% silicon oxide, 24.5% sodium oxide, 24.5% calcium oxide and 6% of phosphorous oxide.

The implant 10 is for example manufactured by machining operations performed on a block. The implant 10 is for example shaped by a method of injection moulding.

The implant 10 can be manufactured in different dimensions depending on the required shape of the implant and its osseointegration, bioabsorption and mechanical properties. The implant 10 can be adapted to male and female patients of all sizes, in order to fill or reconstruct any type of bone.

The implant 10 is preferably attached. The implant 10 is for example attached with screws or with glue to a bone of the person who receives the implant 10. The implant 10 is, for example, attached to cartilage. The implant 10 can be attached to a bone and/or to cartilage. Any other combination enabling the attachment of the implant 10 is possible.

The implant 10 has a material density by volume preferably ranging from 20% to 100%, and more preferably from 50% to 80%. For example, a density of 100% corresponds to an implant 10 without porosity, i.e. entirely made of ceramic material. For example, an implant 10 with a porosity of 60% by volume is made of, in terms of volume, 60% of ceramic material and of 40% of absence of ceramic material. The absence of ceramic material corresponds to the macroporosity of the implant 10.

Interconnected macroporosity is preferably present in the porous portion 3. For example, the dense portion 1 requires mechanical properties ensuring the implant is properly supported as it undergoes constraints of everyday life, and the porous portion 3 enables proper osseointegration and/or good bioresorbtion so that the implant becomes secured to the bone. Preferably, the implant features a good ratio of the dense portion 1 to the porous portion 3 so that osseointegration of the porous portion is quick and enables a proper connection of the dense portion to the bone on which it is implanted, so that the dense portion has mechanical properties that are similar to, and even better than that of the original bone. The dense portion, depending on its dimensions, will undergo varying bioresorbtion/biointegration speeds.

Preferably, the thickness of the porous portion 3 varies from 0.5 mm to 10 mm. Preferably, the porous portion 3 is thinner at its ends and thicker in its centre, as shown in Figs. 1c, 2c and 3c.

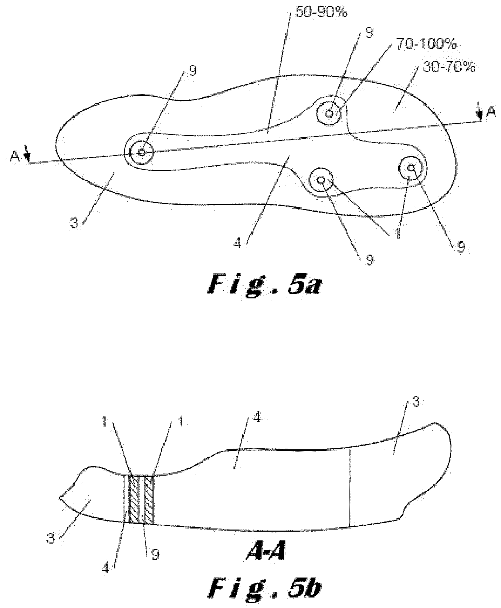

Fig. 5a shows an implant 10 comprising four dense portions 1, and an intermediate portion 4 mechanically coupling the four dense portions 1. The intermediate portion 4 is mechanically coupled to a porous portion 3. This embodiment combines good bioresorbtion properties with good mechanical properties. The transfer of constraints occurs preferentially from the dense portions 1 towards the porous portion 3, passing through the intermediate portion 4. The dense portions 1 comprise attachment holes 9 or holes intended for the fixing of the implant 10. Fig. 5b shows a cross-section view along the axis A-A of Fig. 5a. The porous portions 3 preferably comprise a portion of the porous portion that is peripheral to the implant and that has a thickness that is thinner than the thickness of the porous portion 3 in contact with the intermediate portion 4, so as to provide improved short-term biointegration properties.

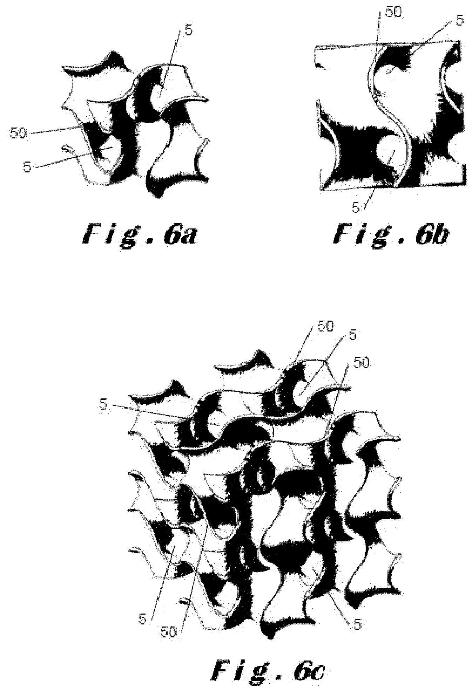

Figs. 6a and 6b show a same basic pattern of the gyroid type, under different viewing angles, i.e. defining a triply periodic minimal surface (more broadly than gyroid). For example, the basic pattern of Figs. 6a to 6c is defined, in a three-dimensional space (x, y, z) with the equation cos *x* . sin *y* + cos *y* . sin *z* + cos *z* . sin *x =* 0, which provides a non-minimal surface that nonetheless has a result that is very close to that of an actual gyroid. Fig. 6c shows a set of eight basic patterns such as that shown in Figs. 6a and 6b. Thus, the creation of a network of basic patterns make it possible to achieve a porosity comprising cavities with well-controlled sections, without changing the dimensions of the cavity sections at the level of the connections between the basic patterns and the walls of the cavities (or side walls 50) and without an abrupt change of the orientation of the cavity walls. The side walls 50 that do not feature an abrupt change of their orientation are said to be non-self-intersecting. Such a network of basic patterns enables the creation of a network of cavities, all interconnected with one another. The advantage of such a network of basic patterns is that it changes the volume density of a portion of the implant 10 comprising such a network of basic patterns, for example by changing the thickness of the cavity walls for a same dimension of the basic pattern. Another advantage of having such a network of basic patterns for the creation of the implant is that it changes the number of cavities per unit of volume, by varying the dimensions of the implant, while retaining the volume density of the portion of the implant 10. Preferably, a portion of the implant formed by the network of basic patterns according to the invention comprises cavity walls 50, 42, 12.

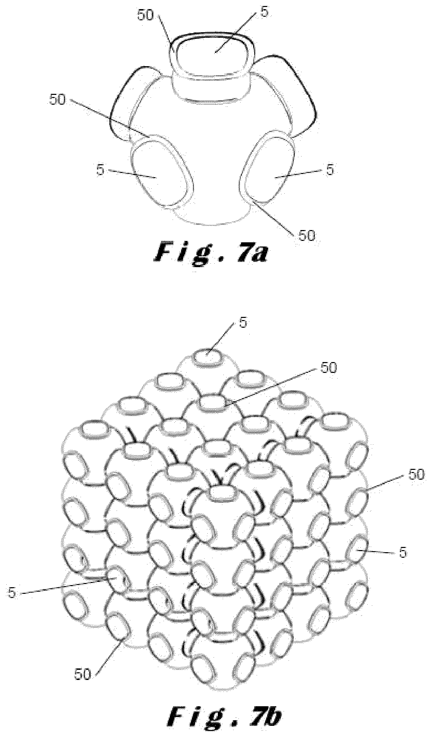

Figs. 7a and 7b show in Fig. 7a a basic pattern intended to form a portion of the implant such as shown in Fig. 7b. Fig. 7a shows a basic pattern comprising a hollow sphere with six orifices enabling the creation of a network of basic patterns according to Fig. 7b in a three-dimensional space. An advantage of this basic pattern and of the network is that it creates a double network of cavities, each cavity network featuring three-dimensional interconnections.

A method for manufacturing the implant 10 using a 3D printing technique enables the manufacturing of an implant 10 from a printing material. This embodiment of the invention relies on the availability of a 3D printer depositing the printing material in a controlled manner. The 3D printer, for example, deposits thin uniform layers of the printing material. Furthermore, the 3D printing machine has a light source in which the wavelength enables the light curing of the material to be printed as well as an optical projection system exposing the printing material during its deposition. The printing material, when exposed to the light source, is cured thanks to the presence of photoinitiators and monomers or polymers able to react with the photoinitiators present in the composition of the printing material.

The light curing of a first layer of the printing material deposits on it a second layer of printing material. The successive curing of the deposited layers, following a well-defined geometry for each one of the layers, enables the manufacturing of the implant 10 according to the invention.

The geometry of the printed layers is defined by a program that breaks the object down into slices. This program, for example, defines the thickness of the printed slices. A breakdown into slices of reduced thickness provides a better level of detail of the finished product. A breakdown into slices of increased thickness provides a reduced level of detail of the finished product. The number of slices the facial implant is broken down into is selected based on the manufacturing time and the required level of detail, in particular. Thicker slices require a longer exposure of the layer to the source of light or exposure to a source of light that delivers increased light intensity.

The printing material is, preferably, a formulation highly enriched in inorganic materials in the form of particles. The particles are preferably enriched in hydroxyapatite and tricalcium phosphate. The light-curing polymer-based material and the photoinitiator bonds the particles of inorganic materials in order to achieve a printing material with no inclusion of air. The printing material preferably has a relatively high viscosity, preferably ranging from 0.01 Pa.s to 1000 Pa.s to ensure that it remains in place before and during the light-curing step.

After the printing and curing of the different layers, any non-cured printing material is removed from the printed item. This step is for example conducted by immersing the printed item in a solvent bath. This step can further be completed by a thermal treatment.

After the printing and curing of the various layers, the ceramic particles are preferably compacted with one another.

The manufacturing of the implant 10 by an additive manufacturing method can be conducted by stereolithography.

The manufacturing of the implant 10 by an additive manufacturing method can be conducted by binder jetting, i.e. the deposition of successive layers of a binder on a powder bed. The powder bed is made of particles of synthetic bioceramic for example.

For example, an additive manufacturing method of ceramic integrates materials, manufacturing machines and designs such that:
- The machine virtually divides the 3D file into a succession of very thin layers.
- The printer then spreads a material layer (25 to 100 µm) and a UV source (DLP) is simultaneously activated to harden the material.
- The machine then spreads a new layer on top of the first one, hardens the material, and repeats this step until the object is fully manufactured.
- At the end of the process, the object is retrieved and excess material is removed.
- To obtain objects made purely of ceramic, the resin has to be eliminated and the powder must be compacted. The parts are placed in an oven to burn off the resin (debinding step). The grains of ceramic are bonded to one another by very weak chemical bonds.
- The temperature increase merges the grains of powder at the level of the grain surfaces (sintering step).
- Once removed from the oven, the parts are inspected and their dimensions are checked.

The present invention has been described for specific embodiments, that are provided solely by way of example and cannot be considered as being limited thereto. Generally speaking, the present invention is not limited to the examples provided and/or described above. The use of terms such as "comprise", "include", "feature" or any other variant thereof, and their conjugated forms, are not to be taken to exclude the presence of elements other than those mentioned. The use of an indefinite article "a" or of the definite article "the" to introduce an element does not exclude the presence of a plurality of these elements. The reference numbers in the claims do not limit the scope thereof.

In short, the invention can also be described as follows.

Implant 10 made of a biocompatible ceramic of synthetic origin obtained by additive manufacturing and comprising:
- a dense portion 1 featuring a material density by volume greater than 70%, and
- a porous portion 3 connected to said dense portion 1 by a connection zone 2, said porous portion 3 comprising an average macroporosity characterised by:
   ∘ a material density ranging from 30% to 70% by volume;
   ∘ cavities 5 defining cavity sections, with a diameter ranging from 0.3 mm to 1.2 mm;
   said dense portion 1 and said porous portion 3 defining an external surface 8;
   said cavities 5 open onto said external surface 8.

## Claims

1. Implant (10) made of a biocompatible ceramic of synthetic origin obtained by additive manufacturing, wherein said implant (10)
has a material density by volume ranging from 20% to 100%;
wherein said implant (10) comprises
cavities (5) delimited at least partially by side walls (50) defining cavity sections, said cavity sections each having an extension such that it is possible to fit each of said cavity sections in a circle having a diameter ranging from 0.3 mm to 1.2 mm;
wherein said side walls (50) delimiting at least partially the cavities (5) are defined by a triply periodic minimal surface.

2. Implant (10) according to claim 1, **characterised in that** the side walls (50) delimiting at least partially the cavities (5) are defined by a triply periodic minimal surface of the gyroid type.

3. Implant (10) according to claim 1 or claim 2, **characterised in that** said side walls (50) are smooth.

4. Implant (10) according to the preceding claim, **characterised in that** said smooth side walls (50) are defined by a mathematical function in three dimensions so that they are all non-self-intersecting.

5. Implant (10) according to any one of the preceding claims, **characterised in that** said side walls (50) comprise a single non-intersecting surface.

6. Implant (10) according to any one of the preceding claims, **characterised in that** the cavities (5) are curved and are preferably defined by a radius of curvature ranging from 300 pm to 1000 pm, and yet even more preferably by a radius of curvature ranging from 500 pm to 750 pm.

7. Implant (10) according to any one of the preceding claims, **characterised in that** the cavities (5) open onto an external surface (8) through openings (6).

8. Implant (10) according to the preceding claim, **characterised in that** the cavities (5) all communicate with said external surface (8).

9. Implant (10) according to any one of the preceding claims, **characterised in that** the diameters of the cavities are distributed periodically within the implant (10).

10. Implant (10) according to any one of the preceding claims, **characterised in that** the implant (10) has a uniform porosity **characterized by** a single shape and evenly distributed throughout the volume.

11. Implant (10) according to any one of the preceding claims, **characterised in that** said cavities (5) that have a given cavity section are distributed regularly throughout the implant (10) and that said cavity sections are controlled in terms of their size.

12. Implant (10) according to any one of the preceding claims, **characterised in that** the biocompatible ceramic comprises a composition of calcium phosphate ranging from 0 to 100%.

13. Implant (10) according to any one of the preceding claims, **characterised in that** the biocompatible ceramic comprises an hydroxyapatite composition ranging from 0 to 100%, and more preferably ranging from 50% to 100%, and even more preferably ranging from 55% to 97%.

14. Implant (10) according to any one of the preceding claims, **characterised in that** the biocompatible ceramic comprises a tricalcium phosphate composition ranging from 0 to 100%, and more preferably ranging from 0 to 50%, and even more preferably ranging from 3% to 45%.

15. Implant (10) according to any one of the preceding claims, **characterised in that** the implant (10) is a cranial-maxillofacial implant, preferably selected from the following types: cranial implant, upper jaw bone implant, facial implant.
